# EUROPÄISCHE PATENTSCHRIFT

(11) **EP 1 968 502 B1**
(45) Veröffentlichungstag und Bekanntmachung des Hinweises auf die Patenterteilung: **15.03.2017**
(21) Anmeldenummer: 05850165.1
(22) Anmeldetag: 15.12.2005
(51) Int. Cl.: A61F 2/46

(54) **CHIRURGISCHES WERKZEUG**
SURGICAL TOOL
OUTIL CHIRURGICAL

(43) Veröffentlichungstag der Anmeldung: 17.09.2008
(73) Patentinhaber: Müller, Erich Johann, 63839 Kleinwallstadt (DE)
(72) Erfinder: Müller, Erich Johann, 63839 Kleinwallstadt (DE)
(74) Vertreter: Becker Kurig Straus
(86) Internationale Anmeldenummer: PCT/DE2005/002271
(87) Internationale Veröffentlichungsnummer: WO 2007/068219

(56) Entgegenhaltungen:
- EP-A- 0 408 109
- DE-C1- 4 332 872
- DE-U1- 8 400 642
- DE-U1- 9 418 964
- DE-U1- 29 508 578
- FR-A- 2 742 334
- FR-A- 2 749 501
- US-A- 4 993 410

## Beschreibung

Die Erfindung betrifft ein chirurgisches Werkzeug zum Ausschlagen und/oder Einsetzen von Prothesen, insbesondere Gelenkprothesen, insbesondere Hüft- und/oder Schulterprothesen.

Bekannt sind chirurgische Werkzeuge, die eine Stellvorrichtung mit komplexem Aufbau aus vielen Einzelteilen aufweisen und eine eng daran angeschlossenen Bedienvorrichtung für die Festsetzung der Prothese. Hierdurch erhält der Chirurg nur ein enges Arbeitsfeld und das Einsetzen der Prothese ist fehleranfällig.

Aufgabe der vorliegenden Erfindung ist es, ein chirurgisches Werkzeug bereitzustellen, das eine verbesserte Festsetzung des Werkzeugs an der Prothese und zugleich eine sichere und gezielte Kraftaufbringung auf die Prothese ermöglicht.

Die Aufgabe wird gelöst durch ein chirurgisches Werkzeug zum Ausschlagen und/oder Einsetzen von Prothesen, insbesondere Gelenkprothesen, insbesondere Hüft- und/oder Schulterprothesen, mit einem, insbesondere stetig, gekrümmten Werkzeugkopf, der einen Durchgang zur Aufnahme eines Prothesenhalses und eine Riegeleinrichtung aufweist, die den Prothesenhals im Durchgang lösbar blockiert, wobei die Riegeleinrichtung ein in den Durchgang eingreifendes Sperrglied aufweist, dessen Eingriffsposition an unterschiedliche Durchmesser des Prothesenhalses anpassbar ist, wobei auf das Sperrglied mittels eines Stellglieds eine Stellkraft auszuüben ist, wobei die Betätigung des Stellglieds über ein Stellelement außerhalb des Operationsfeldes mit Abstand zum Sperrglied einzurichten ist.

Die Festsetzung der Prothese in dem Durchgang des Werkzeugs kann durch den Abstand der Stelleinrichtung auf einfache Weise außerhalb des Operationsfeldes erfolgen. Dies ermöglicht ein ungestörtes Arbeiten für den Chirurgen und zugleich wird der Zustand im Operationsfeld nicht beeinflusst oder geändert. Beispielsweise kann auf diese Weise eine nichtzementierte Prothese mit dem Werkzeug eingesetzt werden, wobei dann ein Krafteintrag in Richtung der Prothese erfolgt. Eine nichtzementierte und/oder eine zementierte Prothese kann durch das erfindungsgemäße Werkzeug einfach und sicher gelöst werden.

Aus der DE 4332872C1 ist ein Ausschlagwerkzeug mit einem Werkzeugkopf bekannt, der mittig zur Aufnahme eines Prothesenhalses geöffnet ist. Im Werkzeugkopf sind an der Aufnahme verschiebbare Stellglieder angeordnet, die durch eine anschließende Druckstange verschoben werden können und so einen aufgenommenen Prothesenhals festklemmen können. In DE 29508578 ist ein Werkzeugteil für ein solches Ausschlagwerkzeug offenbart, bei dem Stellglieder durch eine axial verschiebliche Druckstange eingestellt werden können. Der Kopf des Werkzeugteils ist seitlich geschlossen.

FR2742334 offenbart ein Ausziehwerkzeug für Hüftgelenkprothesen, bei dem eine Stellstange in einem Schaft läuft und durch einen auf dem Schaft laufenden und mit der Stange verbundenen Griff verschoben werden kann. Die Stellstange verschiebt ein schräg zur Stange verlaufendes Stellglied im Werkzeugkopf so, dass ein Prothesenhals zwischen Stellglied und einem Endstück eingeklemmt werden kann. Das Werkzeug hat einen seitlich offenen Werkzeugkopf.

FR 2749501 beschreibt ein Ausschlagwerkzeug, bei dem die Prothese im wesentlichen durch ein festziehbares fadenartiges Element gehalten wird, wie etwa ein Drahtseil, ein textiles Element oder ähnliche. Der Prothesenhals wird in eine Schlinge gelegt und gegen ein Haltestück angezogen.

Die Aufgabe wird ebenfalls gelöst durch ein chirurgisches Werkzeug zum Ausschlagen und/oder Einsetzen von Prothesen, insbesondere Gelenkprothesen, insbesondere Hüft- und/oder Schulterprothesen, insbesondere nach Anspruch 1, mit einem insbesondere stetig gekrümmten Werkzeugkopf, der einen Durchgang zur Aufnahme eines Prothesenhalses und eine Riegeleinrichtung aufweist, die den Prothesenhals im Durchgang lösbar blockiert, wobei die Riegeleinrichtung ein in den Durchgang eingreifendes Sperrglied aufweist, dessen Eingriffsposition an unterschiedliche Durchmesser des Prothesenhalses anpassbar ist, wobei auf das Sperrglied mittels eines Stellglieds eine Stellkraft auszuüben ist, wobei der Werkzeugkopf an einer Seite des Durchgangs eine Öffnung zum seitlichen Einführen des Prothesenhalses aufweist.

Durch die seitliche Öffnung können auch Prothesen beaufschlagt werden, die aus einem Block hergestellt sind und keinen abnehmbaren Kopf aufweisen, sogenannte Monoblockprothesen. Darüber hinaus können Prothesen bewegt werden, bei denen es schwer ist, den Kopf zu lösen oder bei denen eine Lösung des Kopfes eine Beschädigung bewirken könnte.

Die Aufgabe wird ebenfalls gelöst durch ein chirurgisches Werkzeug zum Ausschlagen und/oder Einsetzen von Prothesen, insbesondere Gelenkprothesen, insbesondere Hüft- und/oder Schulterprothesen, insbesondere nach Anspruch 1 oder 2, mit einem insbesondere stetig gekrümmten Werkzeugkopf, der einen Durchgang zur Aufnahme eines Prothesenhalses und eine Riegeleinrichtung aufweist, die den Prothesenhals im Durchgang lösbar blockiert, wobei die Riegeleinrichtung ein in den Durchgang eingreifendes Sperrglied aufweist, dessen Eingriffsposition an unterschiedliche Durchmesser des Prothesenhalses dadurch anpassbar ist, dass ein seitlich geöffnetes, zumindest teilweise zusammenpressbares Einsetzelement mit einer im wesentlichen zentralen Aufnahme zum Halten eines Prothesenhalses in dem Durchgang des Werkzeugkopfes eingesetzt ist.

Durch das Einsetzelement in dem Durchgang des Werkzeugkopfes können auch leicht zu beschädigende Prothesen sicher aus der Einsetzbefestigung entfernt bzw. eingesetzt werden. Durch das Einsetzelement wird die aufgebrachte Kraft allseitig gleichmäßig verteilt und es führt zur Verminderung von Spannungsspitzen insbesondere im Bereich des Sperrgliedes. Der Innendurchmesser des Einsetzelements kann leicht an den Durchmesser des Prothesenhalses angepasst werden, wobei der Außendurchmesser des Einsetzelements im wesentlichen gleich bleiben kann, angepasst an den Durchmesser des Werkzeugdurchgangs. Das Einsetzelement kann dabei in Durchgänge mit nahezu beliebiger Form eingesetzt werden, wobei lediglich auf eine an die Prothesenhalsform angepasste Aufnahmeform zu achten ist.

Vorteilhaft ist es, wenn das seitlich geöffnete Einsetzelement so in den Durchgang des seitlich geöffneten Werkzeugkopfes eingesetzt ist, dass die Öffnungen korrespondieren. Der Werkzeugkopf kann auf diese Weise auch für preisgünstigere bzw. ältere Monoblockprothesen verwendet werden, aber auch für alle anderen Prothesen, die ebenfalls leichter in die Öffnung des Werkzeugkopfes und des Einsetzelements einzusetzen sind und zugleich sicher und beschädigungsfrei gehalten werden.

Vorteilhaft ist es, wenn die Aufnahme des Einsetzelements konisch zulaufend ausgebildet ist und einen an den jeweiligen Durchmesser eines Prothesenhalskonusses angepassten Innendurchmesser aufweist. Der Prothesenhals weist regelmäßig einen leicht konkav ausgebildeten Prothesenhalskörper und einen daran anschließenden konisch zulaufenden Prothesenhalskonus auf, auf den der Prothesenkopf aufgesteckt werden kann. Durch die konisch zulaufende Form der Aufnahme des Einsetzelements ist der Prothesenhalskonus sicher allseitig umschlossen und die Prothese kann auch am Prothesenhalskonus bewegt werden.

Vorteilhaft ist es, wenn das Stellglied eine Stellstange ist, die an einem werkzeugkopfabgewandten Ende über das Stellelement zu betätigen ist, wobei das Stellelement zum Vorschub und zur lösbaren Blockierung des Sperrglieds in eine Verriegelungsposition im Werkzeugkopf einzusetzen ist.. Durch die Verwendung der Stellstange kann zum einen ein großer Abstand zum Werkzeugkopf eingehalten werden und zugleich wird die Ausübung der Stellkraft einfach und sicher.

Eine leicht anpassbare und variable einstellbare Stellvorrichtung liegt vor, wenn das Stellelement durch einen Hebel mit einem exzentrisch ausgebildeten Hebelkopf gebildet ist, der auf das werkzeugkopfabgewandte Ende der Stellstange wirkt und eine vom Schwenkwinkel abhängige axiale Vorschubposition des Sperrglieds definiert.

Die Stellkraftweiterleitung ist besonders zuverlässig und sicher, wenn zwischen Hebelkopf und werkzeugkopfabgewandten Ende der Stellstange ein elastisches und zugleich kraftübertragendes Kontaktelement, insbesondere aus Kunststoff, angeordnet ist. Der Kunststoff wird dabei vorzugsweise elastisch gewählt, da somit ein sicherer und dauerhafter Kontakt zwischen den Elementen hergestellt ist und zugleich wird das Kontaktelement so hart sein, dass eine ausreichende Kraftübertragung zwischen Hebel und Stellstange möglich ist.

Eine leichte und zuverlässige Einstellbarkeit des Abstandes zwischen Werkzeugkopf und Stellelement kann vorgenommen werden, wenn das Stellelement ein Stellgehäuse ist, das ein Stellgewinde aufweist, durch das der Abstand zwischen Werkzeugkopf und Stellgehäuse einzustellen ist. Durch das Stellgewinde können auch größere Strecken bei der Einstellung des Werkzeugs überwunden werden, wobei in jedem Zwischenzustand ein sicherer Halt der Elemente des Werkzeugs durch den Eingriff der Gewinde gegeben ist.

Vorteilhaft ist es, wenn der Hebelkopf innerhalb und/oder an dem Stellgehäuse angeordnet ist. Die Gewindeeinstellung bewirkt dann die größere Abstandseinstellung des Stellelements und die Hebelkippung bewirkt lediglich die letzte, wesentlich kürzere Abstandseinstellung bis zum vollständigen Kontakt des Stellelements zum Sperrglied. Insbesondere falls der Abstand bereits bei Einsetzen des Prothesenhalses in den Durchgang des Werkzeugkopfes sehr gering ist, kann auch die alleinige Einstellung des Stellkontakts durch den Hebel ausreichen.

Vorteilhaft ist es, wenn ein Führungsgehäuse, durch das hindurch die Stellstange verschieblich geführt ist, an einem werkzeugkopfzugewandten Ende ein Gewinde zur Verbindung von Führungsgehäuse und Werkzeugkopf aufweist.

Vorteilhaft ist es, wenn ein Führungsgehäuse an einem werkzeugkopfabgewandten Ende ein Stellgewinde als Verbindung zu dem Stellgehäuse aufweist, insbesondere zur Feinjustierung im Sinne eines zusätzlichen Stellelements.

Eine einfache und sichere Bedienbarkeit des Werkzeugs ist gegeben, wenn das Führungsgehäuse an einem werkzeugkopfabgewandten Ende ein Gewinde als Verbindung zu einem Handgriff aufweist, wobei der Handgriff über ein Stellgewinde zur Abstandseinstellung mit dem Stellgehäuse verbunden ist.

Vorteilhaft ist es, wenn das Führungsgehäuse als Handgriff dient.

Vorteilhaft ist es, wenn das Führungsgehäuse auf einem Außendurchmesser einen verschieblichen Schlaggriff aufweist, der insbesondere zwischen Werkzeugkopf und Stellgehäuse zu verschieben ist.

Eine sicher reproduzierbare Kraftausübung ist möglich, wenn die Schlagkraft auf das im Werkzeugkopf an die Prothese angestellte Sperrglied durch ein vorzugsweise an einen Adapter an dem Stellgehäuse ansetzbares Powertool auszuüben ist. Die Kraft und die Schlagfrequenz kann durch das Powertool einfach eingestellt werden, beispielsweise angepasst an die Größe und die Belastbarkeit der Prothese. Darüberhinaus ist das Powertool auch für andere Einsatzzwecke zu verwenden, so dass eine Mehrfachnutzung und somit eine beträchtliche Kostenersparnis vorliegt. Das Powertool kann dabei beispielsweise pneumatisch oder elektrisch arbeiten.

Ein sehr kompaktes Werkzeug liegt vor, wenn die Schlagkraft durch Entspannen einer Spannfeder, die im Führungsgehäuse untergebracht ist, auszuüben ist, wobei die Spannfeder über ein verriegelbares und entriegelbares Schlag/Haltelement zu spannen ist und nach dem Entriegeln das Schlag/Haltelement durch die Federkraft gegen ein werkzeugkopfabgewandtes Ende zum Lösen der Prothese oder gegen ein werkzeugkopfzugewandtes Ende zum Einsetzen der Prothese schlägt. Die Feder ist dabei auch bei großer Federkraft sicher im Führungsgehäuse untergebracht und kann zu keiner Verletzung führen. Darüberhinaus kommt das Schlagelement in dieser Form nicht mit dem Operationsbereich in Kontakt. Die Stärke und die Richtung der Federkraft kann leicht durch Austausch bzw. Umsetzen der Feder erreicht werden.

Eine einfaches und sicheres Spannen ist möglich, wenn die Spannfeder mittels Bewegung des angrenzenden Schlag/Haltelements durch eine damit verbundene Zahnstange und über ein darauf laufendes Zahnrad, das an dem Führungsgehäuse gelagert ist, zu spannen ist. In einer anderen Variante kann die Spannung der Feder beispielsweise durch einen Schrittmotor vorgenommen werden.

Eine sehr einfaches und zuverlässiges Werkzeug liegt vor, wenn die Schlagkraft durch ein stoßartiges Anschlagen eines Schlagelements auszuüben ist, das auf dem Führungsgehäuse verschieblich geführt ist, gegen ein werkzeugkopfabgewandtes Ende des Führungsgehäuses und/oder ein weiteres, damit verbundenes Element zum Lösen der Prothese oder gegen ein werkzeugkopfzugewandtes Ende des Führungsgehäuses zum Einsetzen der Prothese. Die Richtung der Kraftausübung kann sehr leicht geändert werden, ohne dass eine Änderung am Werkzeug vorgenommen werden müsste.

Vorteilhaft ist es, wenn das Sperrglied durch im Werkzeugkopf gebildete gegenüberliegende Nuten geführt ist. Hierdurch ist das Sperrglied sicher gehalten und zugleich leicht verschiebbar, so dass eine einfache Anpassung an den Prothesenhals möglich ist.

Vorteilhaft zur Anpassung an kleine Prothesen ist es, wenn das Sperrglied länger ausgebildet ist, so dass schmalere Prothesenhälse, insbesondere zwischen etwa 8 bis 12 mm festzusetzen sind.

Vorteilhaft zur Anpassung an größere Prothesen ist es, wenn das Sperrglied kürzer ausgebildet ist, so dass breitere Prothesenhälse, insbesondere zwischen etwa 11 bis 16 mm festzusetzen sind.

Die Aufgabe wird ebenfalls gelöst durch ein Verfahren zum Einsetzen und/oder Entfernen einer Prothese mittels eines Werkzeugs insbesondere nach einem der Ansprüche 1 bis 22, wobei um einen Prothesenschaft ein Werkzeugkopf mit einem Durchgang gesetzt wird, der über ein Sperrglied an dem Prothesenhals festgesetzt wird, wozu ein Stellglied über ein Stellelement und/oder ein Stellgewinde an das Sperrglied angesetzt wird und eine Schlagkraft auf die Prothese ausgeübt wird, insbesondere über ein Schlagelement und/oder ein Führungsgehäuse mit einer Spannfeder oder über ein Powertool.

Weitere Merkmale und Vorteile der Erfindung ergeben sich aus den Ansprüchen und der nachstehenden Beschreibung, in der Ausführungsbeispiele des Gegenstands der Erfindung in Verbindung mit den Zeichnungen näher erläutert sind.

Es zeigen:
- Fig. 1: ein chirurgisches Werkzeug im Längsschnitt,
- Fig. 2: ein chirurgisches Werkzeug in Explosionsdarstellung,
- Fig. 3: ein chirurgisches Werkzeug in perspektivischer Darstellung,
- Fig. 4: ein chirurgisches Werkzeug im Längsschnitt,
- Fig. 5a: einen Werkzeugkopf,
- Fig. 5b: einen Werkzeugkopf im Schnitt,
- Fig. 6a: einen Werkzeugkopf in Aufsicht,
- Fig. 6b: ein Einsetzelement in Aufsicht,
- Fig. 6c: ein Einsetzelement im Längsschnitt
- Fig. 7a: ein chirurgisches Werkzeug mit Feder,
- Fig. 7b: ein chirurgisches Werkzeug mit geöffneter Seitendarstellung und
- Fig. 7c: ein chirurgisches Werkzeug im Querschnitt.

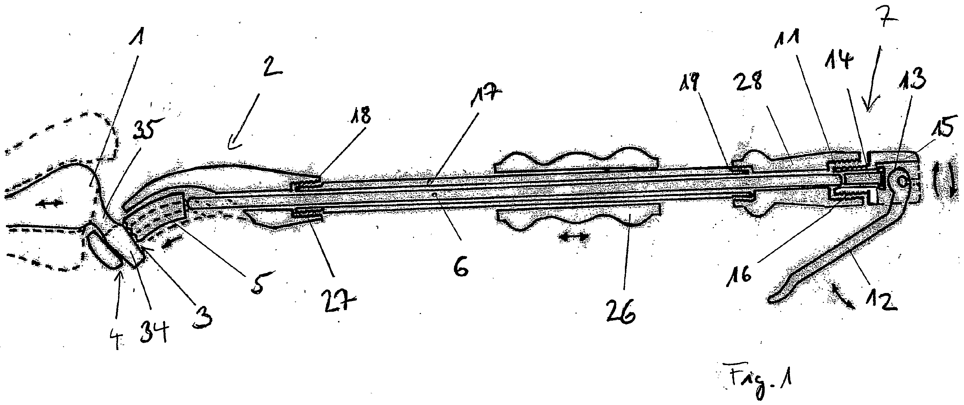

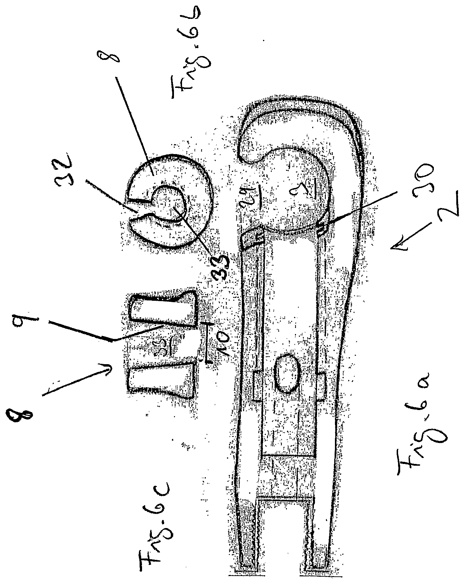

Fig. 1 zeigt ein chirurgisches Werkzeug im Längsschnitt. Das chirurgische Werkzeug weist einen Werkzeugkopf 2 auf, in dessen Durchgang 3 ein Hals 4 einer Prothese 1 eingeführt ist. Durch ein Sperrglied 5, das im Werkzeugkopf 2 verschieblich eingesetzt ist, wird der Prothesenhals 4 festgesetzt. Insbesondere ist bei Verwendung eines Einsetzelements 8 mit einer konischen Aufnahme 33, wie in Fig. 6b dargestellt, ein Einsetzen eines Prothesenhalskonusses 34 möglich. Der Durchgang 3 kann jedoch auch an den konkav geformten Prothesenhalskörper 35 angesetzt werden, wenn ausreichend Platz vorhanden ist und der Bereich ausreichend Stabilität aufweist. Das Sperrglied 5 wird durch ein Stellglied 6, hier in Form einer Stellstange, so weit vorgeschoben, dass die Prothese im Durchgang 3 fest sitzt. Das Stellglied 6 ist hierzu in einem am Werkzeugkopf 2 mittels eines Gewindes 27 angesetzten Führungsgehäuse 17 verschieblich geführt. An einem werkzeugkopfabgewandten Ende 19 weist das Führungsgehäuse 17 einen Griff 28 auf, der wiederum zentral die Stellstange führt bis zu einem Stellgehäuse 15, das sich an den Griff 28 anschließt. Die Verbindung zwischen Griff 28 und Stellgehäuse 15 wird über ein Stellgewinde 16 hergestellt, das zudem eine Einstellung des Abstandes zwischen Stellgehäuse 15 und Werkzeugkopf 2 ermöglicht.

In dem Stellgehäuse 15 ist ein Stellelement 7 in Form eines Hebels 12 mit einem im Stellgehäuse 15 angesetzten exzentrischen Hebelkopf 13 angebracht. Durch Schwenken des Hebels 12 übt der Hebelkopf 13 über ein Kontaktelement 14 eine Zustellkraft auf die Stellstange und somit auf das damit beaufschlagte Sperrglied 5 aus. Mittels des Stellgewindes 16 wird der Abstand zwischen Stellstange und Sperrglied 5 so eingestellt, dass durch Schwenken des Hebels 12 der Stellkontakt zum Sperrglied 5 hergestellt wird, so dass das Sperrglied 5 den Prothesenhals im Durchgang 3 des Werkzeugkopfes 2 festsetzt. Die Prothese ist somit von dem Werkzeug fest gehalten und andererseits befindet sich keine der Stellvorrichtungen im Operationsbereich, wo es ansonsten zu einer Beeinträchtigung des Operationserfolgs kommen könnte.

Auf dem Führungsgehäuse 17 wird ein Schlagelement 26 verschieblich gelagert, das zum Ausüben einer Schlagkraft gegen ein Ende des Führungsgehäuses 17 geeignet ist. Die Schlagkraft wird über die starre Verbindung zwischen Führungsgehäuse 17 und Werkzeugkopf 2 an die Prothese 1 weitergeleitet, die somit je nach Kraftrichtüng eingesetzt oder gelöst werden kann. Wenn das Schlagelement 26 gegen das werkzeugkopfabgewandte Ende 19 des Führungsgehäuses 17 bzw. gegen einen damit verbundenen Anschlag geschlagen wird, wird die Prothese gelöst. Wenn das Schlagelement 26 gegen das werkzeugkopfzugewandte Ende 18 des Führungsgehäuses 17 bzw. gegen einen damit verbundenen Anschlag geschlagen wird, wird die Prothese eingesetzt.

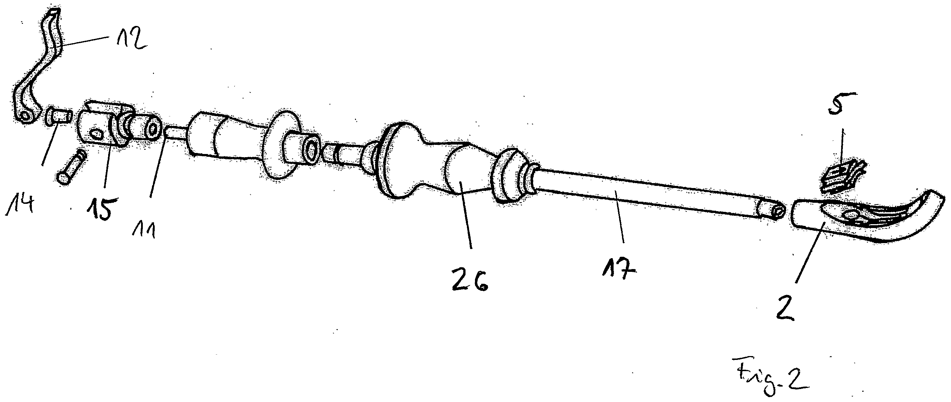

Fig. 2 zeigt ein chirurgisches Werkzeug in Explosionsdarstellung. Die Komponenten des Werkzeugs lassen sich leicht über mehrerer Gewinde aneinander verschrauben, so dass eine sichere Kraftweiterleitung vorgenommen werden kann bzw. das Werkzeug einfach gereinigt werden kann. Das Sperrglied 5 kann aus dem Werkzeugkopf durch einfaches Verschieben in den seitlichen Nuten herausgenommen und gesäubert werden.

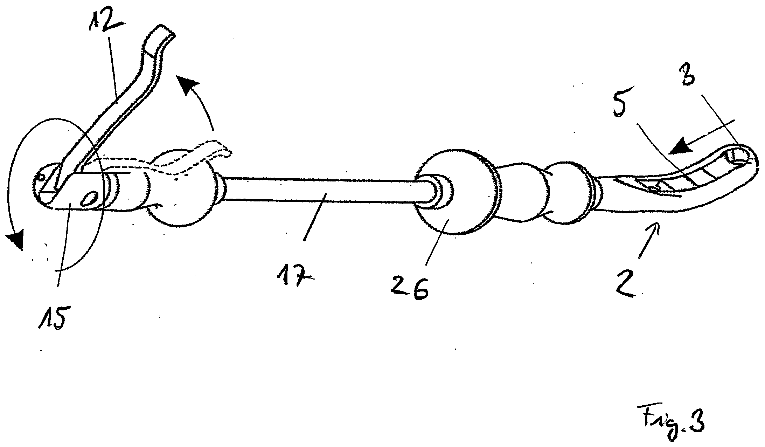

Fig. 3 zeigt ein chirurgisches Werkzeug in perspektivischer Darstellung. Das Ansetzen des Werkzeugs sowie das Ausüben der Kraft erfolgt durch wenige Schritte.
Zunächst wird der Hebel 12 soweit entspannt, dass die Stellstange das Sperrglied 5 freigibt und das Sperrglied 5 den Durchgang 3 im Werkzeugkopf 2 im wesentlichen freilässt. Anschließend wird der Prothesenhals 4 in den Durchgang 3 eingeführt. Durch das Stellgewinde am Stellgehäuse 15 wird die Stellstange weit an das Sperrglied 5 herangeführt. Das Festsetzen der Stellstange erfolgt dann durch einen Ausklappen des Hebels 12, wodurch der exzentrische gelagerte Hebelkopf über das Kontaktelement 14 die Stellstange an das Sperrglied 5 anstellt, das wiederum an den Prothesenhals 4 angesetzt ist. Nun kann die Schlagkraft mit dem Schlagelement 26, das auf dem Führungsgehäuse 17 geführt ist, in der gewünschten Richtung ausgeübt werden.

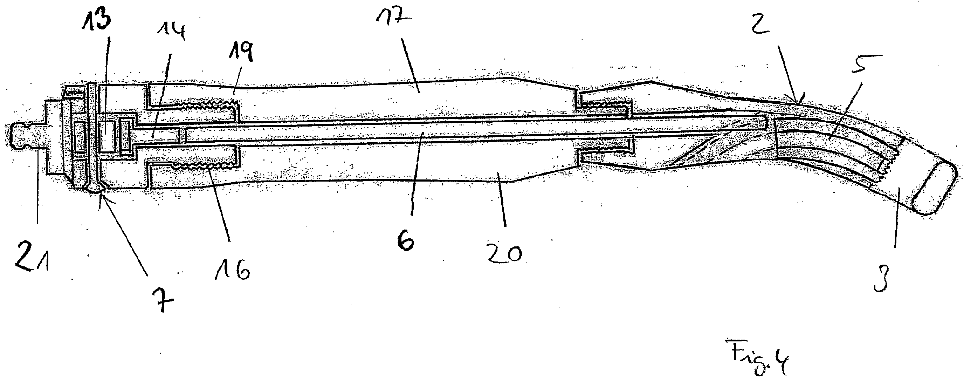

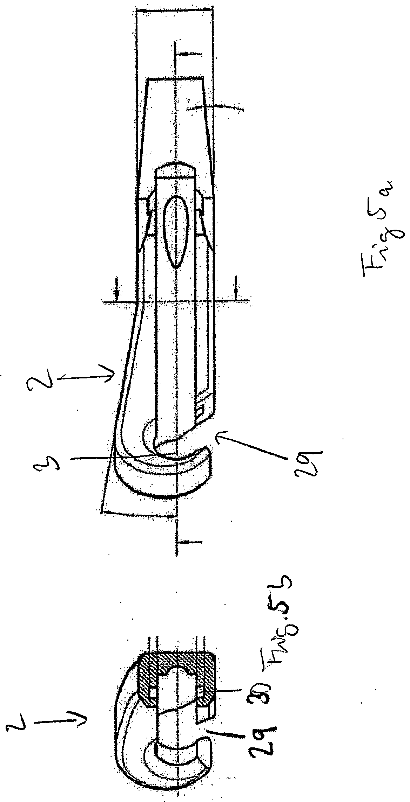

Fig. 4 zeigt ein chirurgisches Werkzeug im Längsschnitt. Das Werkzeug weist einen Werkzeugkopf 2 auf mit einem Sperrglied 5 und einem Durchgang 3. An den Werkzeugkopf 2 ist mittels eines Gewindes ein Führungsgehäuse 17 angebracht, das zugleich als Handgriff 20 dient. Am werkzeugkopfabgewandeten Ende 19 des Führungsgehäuses 17 ist mittels eines Stellgewindes 16 ein Stellgehäuse 15 mit einem Stellelement 7 in Form eines nicht dargestellten Hebels mit einem Hebelkopf 13 angebracht. Dieser Hebelkopf 13 steht wiederum über ein Kontaktelement 14 mit einem Stellglied 6 in Form einer Stellstange in Kontakt, die das Sperrglied 5 im Werkzeugkopf 2 an dem Prothesenhals festsetzt. Zum Aufbringen der Schlagkraft weist das Stellgehäuse 15 einen Adapter 21 auf, an den ein nicht dargestelltes Powertool anbringbar ist, durch das eine Schlagkraft vorzugsweise in zwei unterschiedliche Richtungen auf das Werkzeug aufbringbar ist, wodurch somit zum einen ein Lösen einer Prothese aber auch ein Festsetzen einer Prothese erfolgen kann. Fig. 5a zeigt einen Werkzeugkopf 2 mit einer seitlichen Öffnung 29, durch die ein Prothesenhals 4 in den Durchgang 3 einführbar ist. Dies ist insbesondere bei Prothesen wichtig, die einstückig hergestellt sind, sogenannte Monoblockprothesen, was insbesondere bei älteren oder preisgünstigen Prothesen der Fall ist.
Fig. 5b zeigt einen Werkzeugkopf 2 im Schnitt, wodurch die Öffnung 29 deutlich erkennbar ist. Der Werkzeugkopf 2 weist Nuten 30 auf, in die das entsprechend geformte Sperrglied einschiebbar ist.
Fig. 6a zeigt einen Werkzeugkopf 2 in Aufsicht, der eine seitliche Öffnung 29 aufweist, durch die ein Prothesenhals 4 in den Durchgang 3 einführbar ist. Der Werkzeugkopf 2 weist wiederum Nuten 30 zum Einschieben des Sperrglieds 5 auf.

Fig. 6b zeigt ein Einsetzelement 8 in Aufsicht, das in den Durchgang 3 eines Werkzeugkopfes 2 beispielsweise nach Fig. 6a einsetzbar ist und eine gleichmäßige Kraftverteilung ermöglicht. Der Durchgang 3 muss dabei nicht notwendigerweise eine seitliche Öffnung 29 aufweisen, jedoch erleichtert diese das Einführen des Prothesenhalses 4. Das Einsetzelement 8 weist eine seitliche Öffnung 32 auf und einen Innendurchmesser 10 seiner Aufnahme 33, der an die Prothesenhälse 4 entsprechend angepasst ausgewählt werden kann.
Fig. 6c zeigt ein Einsetzelement 8 nach Fig. 6b im Längsschnitt. Das Einsetzelement 8 weist eine konisch zulaufende Innenfläche 9 auf, wodurch eine gute Anpassung an konisch zulaufende Prothesenhalskonusse 34 möglich ist.

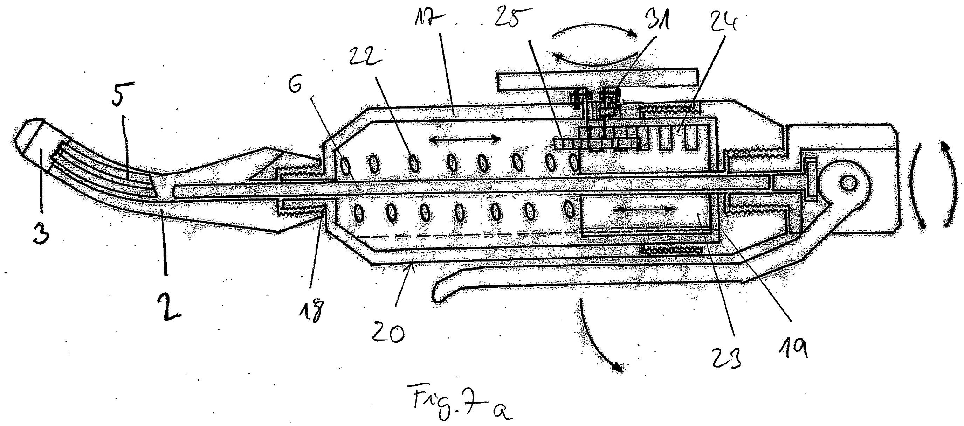

Fig. 7a zeigt ein chirurgisches Werkzeug mit einer Spannfeder 22. Die Spannfeder 22 ist in einem Führungsgehäuse 17 eingesetzt und wird durch ein Schlag/Halteelement 23 an einer Seite beaufschlagt. Dieses Schlag/Halteelement 23 kann durch ein Drehen eines Zahnrades 25, das im Ausführungsbeispiel nur halb ausgeführt ist, auf eine Zahnstange 24, die am Schlag/Halteelement 23 befestigt ist, gespannt werden. Dabei rastet in jeder Drehsituation ein Rastelement 31 ein und hält das Zahnrad 25 in seiner Drehposition so lange bis das Halbrad an die Seite gedreht wird, an der keine Zähne vorhanden sind. Dies führt dazu, dass das Rastelement 31 keinen Gegenhalt mehr hat und das Schlag/Halteelement 23 durch die Federkraft gegen ein werkzeugkopfabgewandtes Ende 19 geschlagen wird. Die hierdurch ausgeübtes Kraft wird über das Führungsgehäuse 17 an den Werkzeugkopf 2 weitergegeben, und von diesem an die Prothese, die durch den Schlag gelöst wird. Wäre die Feder in dem Führungsgehäuse so angesetzt, dass das Schlag/Halteelement 23 an dem anderen Ende des Führungsgehäuses 17 anschlüge, würde eine Einsetzkraft für das Einsetzen einer Prothese erzeugt. Das Werkzeug mit der Spannfeder 22 im Führungsgehäuse 17 ist sehr kompakt und sicher ausgebildet, und kann auch in engen Operationsfeldern eingesetzt werden.

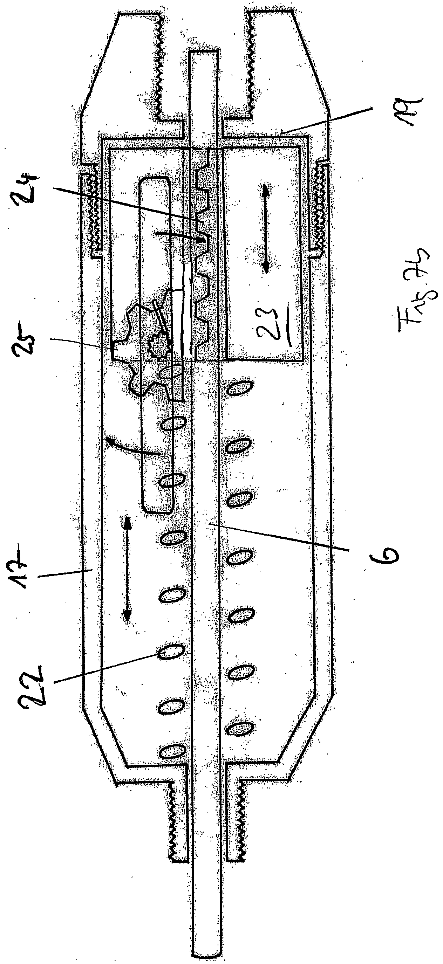

Fig. 7b zeigt ein chirurgisches Werkzeug mit geöffneter Seitendarstellung. Das Zahnrad 25 ist in seitlicher Darstellung gezeigt, wobei die untere Seite des Zahnrades keine Zähne aufweist.

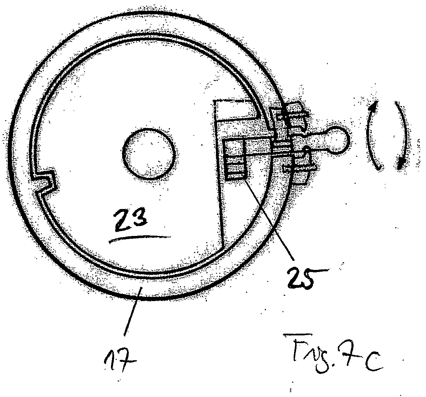

Fig. 7c zeigt ein chirurgisches Werkzeug im Querschnitt, wobei das Zahnrad 25 in eine Ausnehmung des Schlag/Halteelements 23 eingreift.

### Bezugszeichenliste

- 1: Prothese
- 2: Werkzeugkopf
- 3: Durchgang
- 4: Prothesenschaft
- 5: Sperrglied
- 6: Stellglied
- 7: Stellelement
- 8: Einsetzelement
- 9: Innenfläche
- 10: Innendurchmesser
- 11: werkzeugkopfabgewandtes Ende
- 12: Hebel
- 13: Hebelkopf
- 14: Kontaktelement
- 15: Stellgehäuse
- 16: Stellgewinde
- 17: Führungsgehäuse
- 18: werkzeugzugewandtes Ende
- 19: werkzeugkopfabgewandtes Ende
- 20: Handgriff
- 21: Adapter
- 22: Spannfeder
- 23: Schlag/Halteelement
- 24: Zahnstange
- 25: Zahnrad
- 26: Schlagelement
- 27: Gewinde
- 28: Griff
- 29: Öffnung
- 30: Nute
- 31: Rastelement
- 32: Öffnung
- 33: Aufnahme
- 34: Prothesenhalskonus
- 35: Prothesenhalskörper

## Patentansprüche

1. Chirurgisches Werkzeug zum Ausschlagen oder Einsetzen von Gelenkprothesen (1), mit einem stetig gekrümmten Werkzeugkopf (2), der einen Durchgang zur Aufnahme eines Prothesenhalses (4) und eine Riegeleinrichtung aufweist, die den Prothesenhals (4) im Durchgang (3) lösbar blockiert, wobei der Werkzeugkopf (2) an einer Seite des Durchgangs (3) eine Öffnung (29) zum seitlichen Einführen des Prothesenhalses (4) aufweist, wobei die Riegeleinrichtung ein in den Durchgang (3) eingreifendes Sperrglied (5) aufweist, wobei auf das Sperrglied (5) mittels eines Stellglieds (6) eine Stellkraft auszuüben ist, wobei die Betätigung des Stellglieds (6) über ein Stellelement (7) einzurichten ist, welches mit Abstand zum Werkzeugkopf angeordnet ist,
**dadurch gekennzeichnet, dass**
die Eingriffsposition des Sperrglieds an unterschiedliche Durchmesser des Prothesenhalses (4) dadurch anpassbar ist, dass ein seitlich geöffnetes (33), zumindest teilweise zusammenpressbares Einsetzelement (8) mit einer im wesentlichen zentralen Aufnahme (33) zum Halten eines Prothesenhalses (4) in dem Durchgang (3) des Werkzeugkopfes (2) so eingesetzt ist, dass die Öffnungen (29, 32) korrespondieren.

2. Chirurgisches Werkzeug nach Anspruch 1, **dadurch gekennzeichnet, dass** die Aufnahme (33) des Einsetzelements (8) konisch zulaufend ausgebildet ist und einen an den jeweiligen Durchmesser eines Prothesenhalskonus (34) angepassten Innendurchmesser (10) aufweist.

3. Chirurgisches Werkzeug nach einem der Ansprüche 1 oder 2, **dadurch gekennzeichnet, dass** das Stellglied (6) eine Stellstange ist, die an einem werkzeugkopfabgewandten Ende (11) über das Stellelement (7) zu betätigen ist, wobei das Stellelement (7) zum Vorschub und zur lösbaren Blockierung des Sperrglieds (5) in eine Verriegelungsposition im Werkzeugkopf (2) einzusetzen ist.

4. Chirurgisches Werkzeug nach einem der Ansprüche 1 bis 3, **dadurch gekennzeichnet, dass** das Stellelement (7) durch einen Hebel (12) mit einem exzentrisch ausgebildeten Hebelkopf (13) gebildet ist, der auf das werkzeugkopfabgewandte Ende (11) der Stellstange wirkt und eine vom Schwenkwinkel abhängige axiale Vorschubposition des Sperrglieds (5) definiert.

5. Chirurgisches Werkzeug nach einem der Ansprüche 1 bis 4, **dadurch gekennzeichnet, dass** zwischen Hebelkopf (13) und werkzeugkopfabgewandtem Ende (11) der Stellstange ein elastisches und zugleich kraftübertragendes Kontaktelement (14), insbesondere aus Kunststoff, angeordnet ist.

6. Chirurgisches Werkzeug nach einem der Ansprüche 1 bis 5, **dadurch gekennzeichnet, dass** das Stellelement (7) ein Stellgehäuse (15) ist, das ein Stellgewinde (16) aufweist, durch das der Abstand zwischen Werkzeugkopf (2) und Stellgehäuse (15) einzustellen ist.

7. Chirurgisches Werkzeug nach einem der Ansprüche 1 bis 6, **dadurch gekennzeichnet, dass** der Hebelkopf (13) innerhalb und/oder an dem Stellgehäuse (15) angeordnet ist.

8. Chirurgisches Werkzeug nach einem der Ansprüche 1 bis 7, **dadurch gekennzeichnet, dass** das Führungsgehäuse (17), durch das hindurch die Stellstange verschieblich geführt ist, an einem werkzeugkopfzugewandten Ende (18) ein Gewinde zur Verbindung von Führungsgehäuse (17) und Werkzeugkopf (2) aufweist, und/oder dass das Führungsgehäuse (17) an einem werkzeugkopfabgewandten Ende (19) ein Stellgewinde als Verbindung zu dem Stellgehäuse (15) aufweist, insbesondere zur Feinjustierung im Sinne eines zusätzlichen Stellelements, und/oder dass das Führungsgehäuse (17) an einem werkzeugkopfabgewandten Ende (19) ein Gewinde als Verbindung zu einem Handgriff aufweist, wobei der Handgriff über ein Stellgewinde zur Abstandseinstellung mit dem Stellgehäuse verbunden ist und/oder dass das Führungsgehäuse (17) als Handgriff dient.

9. Chirurgisches Werkzeug nach einem der Ansprüche 1 bis 8, **dadurch gekennzeichnet, dass** das Führungsgehäuse (17) auf einem Außendurchmesser einen verschieblichen Schlaggriff (26) aufweist, der insbesondere zwischen Werkzeugkopf (2) und Stellgehäuse (15) zu verschieben ist.

10. Chirurgisches Werkzeug nach einem der Ansprüche 1 bis 9, **dadurch gekennzeichnet, dass** die Schlagkraft auf das im Werkzeugkopf (2) an die Prothese (1) angestellte Sperrglied (5) durch ein vorzugsweise an einen Adapter (21) an dem Stellgehäuse (15) ansetzbares Powertool auszuüben ist und/oder dass die Schlagkraft durch Entspannen einer Spannfeder (22), die im Führungsgehäuse (17) untergebracht ist, auszuüben ist, wobei die Spannfeder über ein verriegelbares und entriegelbares Schlag/Halteelement (23) zu spannen ist und nach dem Entriegeln das Schlag/Halteelement (23) durch die Federkraft gegen ein werkzeugkopfabgewandtes Ende (19) zum Lösen der Prothese oder gegen ein werkzeugkopfzugewandtes Ende zum Einsetzen einer Prothese schlägt und/oder dass die Spannfeder (22) mittels Bewegung des angrenzenden Schlag/Halteelements (23) durch eine damit verbundene Zahnstange (24) und über ein darauf laufendes Zahnrad (25), das an dem Führungsgehäuse (17) gelagert ist, zu spannen ist und/oder dass die Schlagkraft durch ein stoßartiges Anschlagen eines Schlagelements (26) auszuüben ist, das auf dem Führungsgehäuse (17) verschieblich geführt ist, gegen ein werkzeugkopfabgewandtes Ende (19) des Führungsgehäuses (17) und/oder ein weiteres, damit verbundenes Element zum Lösen der Prothese oder gegen ein werkzeugkopfzugewandtes Ende des Führungsgehäuses (17) zum Einsetzen der Prothese.

11. Chirurgisches Werkzeug nach einem der Ansprüche 1 bis 10, **dadurch gekennzeichnet, dass** das Sperrglied (5) durch im Werkzeugkopf (2) gebildete gegenüberliegende Nuten geführt ist und/oder dass das Sperrglied (5) länger ausgebildet ist, so dass schmalere Prothesenhälse, insbesondere zwischen 8 bis 12mm festzusetzen sind und/oder dass das Sperrglied (5) kürzer ausgebildet ist, so dass breitere Prothesenhälse, insbesondere zwischen 11 bis 16 mm festzusetzen sind.

## Claims

1. Surgical tool for knocking out or inserting of joint prostheses (1), with a continuously curved head (2) of the tool, which exhibits a passage for the seating of a prosthesis neck (4) and a bolt mechanism, which detachably blocks the prosthesis neck (4) in the passage (3), wherein the head (2) of the tool exhibits an opening (29) at a side of the passage (3) for the lateral insertion of the prosthesis neck (4), wherein the bolt mechanism exhibits a blocking member (5) intervening in the passage (3), wherein an adjusting force is to be applied to the blocking member (5) by means of an adjustment member (6), where the manipulation of the adjustment member (6) is to be arranged by an adjustment element (7), which is disposed with distance to the head of the tool,
**characterized in that**
the interference position of the blocking element is adaptable to different diameters of the prosthesis neck (4) by the fact that a laterally opened (33), at least partially compressible insertion element (8) with a substantially central seating (33) for holding a prosthesis neck (4) is inserted in the passage (3) of the head (2) of the tool, such that the openings (29, 32) correspond.

2. Surgical tool according to claim 1, **characterized in that** the seating (33) of the insertion element (8) is formed conically tapered and exhibits an inner diameter (10) which is adapted to the respective diameter of a cone (34) of the neck of the prosthesis.

3. Surgical tool according to claim 1 or 2, **characterized in that** the adjustment member (6) is an adjustment rod, which is to be actuated at an end (11), which is facing away from the head of the tool, via the adjustment element (7), wherein the adjustment element (7) is to be inserted into a blocking position in the head (2) of the tool for the heading and for the detachable blocking of the blocking member (5).

4. Surgical tool according to one of the claims 1 to 3, **characterized in that** the adjustment element (7) is formed by a lever (12) with an eccentrically formed head (13) of the lever, which acts upon the end (11) of the adjustment rod, which is facing away from the head of the tool, and which defines an axially heading position of the blocking member (5), which is dependent on a pivoting angle.

5. Surgical tool according to one of the claims 1 to 4, **characterized in that** a contact element (14), especially made of plastic, is disposed between head (13) of the lever and the end (11) of the adjustment rod, which is facing away from the head of the tool, wherein the contact element is elastic and is force-transferring, too.

6. Surgical tool according to one of the claims 1 to 5, **characterized in that** the adjustment element (7) is an adjustment housing (15), which exhibits an adjustment thread (16), by which the distance between the head (2) of the tool and the adjustment housing (15) is adjustable.

7. Surgical tool according to one of the claims 1 to 6, **characterized in that** the head (13) of the lever is disposed in and/or at the adjustment housing (15).

8. Surgical tool according to one of the claims 1 to 7, **characterized in that** the guiding housing (17), through which the adjustment rod is routed relocatably, exhibits at an end (18), which is facing towards the head of the tool, a thread for the connection of the guiding housing (17) and the head (2) of the tool, and/or **in that** the guiding housing (17) at an end (19), which is facing away from the head of the tool, exhibits an adjustment thread as connection to the adjustment housing (15), especially for the fine adjustment in the sense of an additional adjustment element, and/or **in that** the guiding housing (17) at an end (19), which is facing away from the head of the tool, exhibits a thread as connection to a handhold, wherein the handhold is connected to the adjustment housing by an adjustment thread for the adjustment of the distance and/or **in that** the guiding housing (17) serves as a handhold.

9. Surgical tool according to one of the claims I to 8, **characterized in that** the guiding housing (17) at an outer diameter exhibits a relocatable impact grip (26), which is movable especially between head (2) of the tool and adjustment housing (15).

10. Surgical tool according to one of the claims 1 to 9, **characterized in that** the impact force on the blocking member (5), which is engaged to the prosthesis (1) in the head (2) of the tool, is applied by a power tool, which is preferably capable of being adapted at an adapter (21) at the adjustment housing (15), and/or **in that** the impact force is applied by expanding a tension spring (22), which is placed in the guiding housing (17), wherein the tension spring is to be tensioned by an impact/holding element (23), which can be locked and unlocked, and after the unlocking the impact/holding element (23) beats against an end (19), which is facing away from the head of the tool, for releasing the prosthesis or against an end, which is facing towards the head of the tool, for inserting a prosthesis, and/or **in that** the tension spring (22) is to be tensioned by movement of the adjacent impact/holding element (23) by a gear rod (24) connected thereto and by a gear (25) moving thereon, which is supported at the guiding housing (17), and/or **in that** the impact force is applied by impulsive hitting of an impact element (26), which is routed relocatably on the guiding housing (17), against an end (19), which is facing away from the head of the tool, of the guiding housing (17) and/or another element connected thereto for releasing the prosthesis or against an end of the guiding housing (17), which is facing towards the head of the tool, for inserting the prosthesis.

11. Surgical tool according to one of the claims 1 to 10, **characterized in that** the blocking member (5) is guided by opposing grooves, which are formed in the head (2) of the tool, and/or **in that** the blocking member (5) is formed longer, such that smaller prosthesis necks are to be set, in particular between 8 to 12 mm, and/or **in that** the blocking member (5) is formed shorter, such that broader prosthesis necks are to be set, in particular between 11 and 16 mm.

## Revendications

1. Outil chirurgical pour écarter ou introduire des prothèses d'articulation (1), comprenant une tête d'outil (2) courbée de façon constante qui présente un passage pour recevoir un col de prothèse (4) et un dispositif de verrou qui bloque le col de prothèse (4) de façon séparable dans le passage (3), dans lequel la tête d'outil (2) présente sur un côté du passage (3), une ouverture (29) pour l'introduction latérale du col de prothèse (4), dans lequel le dispositif de verrou présente un membre de blocage (5) se mettant en prise dans le passage (3), dans lequel une force de rappel doit être exercée sur le membre de blocage (5) au moyen d'un membre de réglage (6), dans lequel l'actionnement du membre de réglage (6) est à ajuster par un élément de réglage (7) qui est disposé à distance de la tête d'outil,
**caractérisé en ce que**
la position de prise du membre de blocage est adaptable à différents diamètres du col de prothèse (4) **en ce qu'**un élément d'insertion (8) ouvert (33) sur le côté, pouvant être comprimé au moins partiellement, avec une réception (33) essentiellement centrale pour retenir un col de prothèse (4) est ainsi inséré dans le passage (3) de la tête d'outil (2) que les ouvertures (29, 32) correspondent.

2. Outil chirurgical selon la revendication 1, **caractérisé en ce que** la réception (33) de l'élément d'insertion (8) est conçue conique et en pointe et présente un diamètre intérieur (10) adapté au diamètre respectif d'un cône de col de prothèse (34).

3. Outil chirurgical selon la revendication 1 ou 2, **caractérisé en ce que** le membre de réglage (6) est une barre de réglage qui est à actionner par le biais de l'élément de réglage (7) sur une extrémité (11) détournée de la tête d'outil, dans lequel l'élément de réglage (7) est à utiliser pour faire avancer et bloquer de façon séparable le membre de blocage (5) dans une position de verrouillage dans la tête d'outil (2).

4. Outil chirurgical selon l'une des revendications 1 à 3, **caractérisé en ce que** l'élément de réglage (7) est formé par un levier (12) avec une tête de levier (13) formée de manière excentrée, qui agit sur l'extrémité (11) de la barre de réglage détournée de la tête d'outil et définit une position d'avancement axiale du membre de réglage (5) qui est fonction de l'angle de pivotement.

5. Outil chirurgical selon l'une des revendications 1 à 4, **caractérisé en ce qu'**un élément de contact (14) élastique, en particulier en plastique, et transmettant à la fois une force, est disposé entre la tête de levier (13) et l'extrémité (11) de la barre de réglage détournée de la tête d'outil.

6. Outil chirurgical selon l'une des revendications 1 à 5, **caractérisé en ce que** l'élément de réglage (7) est un boîtier de réglage (15) qui présente un filetage de réglage (16) par lequel l'écart entre la tête d'outil (2) et le boîtier de réglage (15) est à régler.

7. Outil chirurgical selon l'une des revendications 1 à 6, **caractérisé en ce que** la tête de levier (13) est disposée à l'intérieur du, et/ou sur le, boîtier de réglage (15).

8. Outil chirurgical selon l'une des revendications 1 à 7, **caractérisé en ce que** le boîtier de guidage (17) à travers lequel la barre de réglage est dirigée en étant mobile, présente sur une extrémité (18) tournée vers la tête d'outil, un filetage pour relier le boîtier de guidage (17) et la tête d'outil (2), et/ou que le boîtier de guidage (17) présente sur une extrémité (19) détournée de la tête d'outil, un filetage de réglage en tant que liaison vers le boîtier de réglage (15), en particulier pour l'ajustement fin au sens d'un élément de réglage supplémentaire, et/ou que le boîtier de guidage (17) présente sur une extrémité (19) détournée de la tête d'outil, un filetage en tant que liaison vers une poignée, dans lequel la poignée est reliée au boîtier de réglage par le biais d'un filetage de réglage pour régler l'écart et/ou que le boîtier de guidage (17) sert de poignée.

9. Outil chirurgical selon l'une des revendications 1 à 8, **caractérisé en ce que** le boîtier de guidage (17) présente sur un diamètre extérieur, une poignée à percussion (26) pouvant être déplacée, qui est à déplacer en particulier entre la tête d'outil (2) et le boîtier de réglage (15).

10. Outil chirurgical selon l'une des revendications 1 à 9, **caractérisé en ce que** la force de percussion sur le membre de réglage (5) disposé sur la prothèse (1) dans la tête d'outil (2) est à exercer par un outil électrique pouvant être mis de préférence sur un adaptateur (21) sur le boîtier de réglage (15) et/ou que la force de percussion est à exercer par la détente d'un ressort de tension (22) qui est disposé dans le boîtier de guidage (17), dans lequel le ressort de tension est à tendre par un élément de percussion/retenue (23) pouvant être verrouillé et déverrouillé, et après le déverrouillage, l'élément de percussion/retenue (23) frappe, par la force de rappel, contre une extrémité (19) détournée de la tête d'outil pour séparer la prothèse ou contre une extrémité tournée vers la tête d'outil pour insérer une prothèse et/ou que le ressort de tension (22) est à tendre au moyen d'un mouvement de l'élément de percussion/retenue (23) adjacent par une crémaillère (24) reliée à celui-ci et par le biais d'une roue dentée (25) circulant dessus qui est disposée sur le boîtier de guidage (17), et/ou que la force de percussion est à exercer par une percussion par à-coups d'un élément de percussion (26) qui est dirigé de façon mobile sur le boîtier de guidage (17), contre une extrémité (19) du boîtier de guidage (17) détournée de la tête d'outil et/ou un autre élément relié à celui-ci pour séparer la prothèse ou contre une extrémité du boîtier de guidage (17) tournée vers la tête d'outil pour insérer la prothèse.

11. Outil chirurgical selon l'une des revendications 1 à 10, **caractérisé en ce que** le membre de réglage (5) est dirigé par des rainures opposées formées dans la tête d'outil (2) et/ou que le membre de réglage (5) est conçu plus long de sorte que des cols de prothèse plus étroits sont à fixer, en particulier de 8 à 12 mm, et/ou que le membre de réglage (5) est conçu plus court de sorte que des cols de prothèse plus larges sont à fixer, en particulier de 11 à 16 mm.
